# EUROPEAN PATENT SPECIFICATION

(11) **EP 4 330 662 B1**
(45) Date of publication and mention of the grant of the patent: **03.09.2025**
(21) Application number: 22725757.3
(22) Date of filing: 26.04.2022
(51) Int. Cl.: G01N 30/34, G01N 30/88, G01N 33/00, G01N 33/15

(54) **ANALYTICAL HPLC**
ANALYTISCHE HPLC
HPLC ANALYTIQUE

(30) Priority: 26.04.2021 GB 202105950
(43) Date of publication of application: 06.03.2024
(73) Proprietor: GE Healthcare Limited, Chalfont St. Giles, Buckinghamshire HP8 4SP (GB)
(72) Inventor: CLARKE, Alan, Nydalen 0401 Oslo (NO); ENGELBRECHT, Hendrik, Petrus, Johnson City, Tennessee 37604 (US); GRIGG, Julian, St. Giles Buckinghamshire HP8 4SP (GB); KHAN, Imtiaz Ahmed, St. Giles Buckinghamshire HP8 4SP (GB); WIKENE, Kristine, Nydalen 0401 Oslo (NO); MCROBBIE, Graeme, St. Giles Buckinghamshire HP8 4SP (GB)
(74) Representative: Coles, Andrea Birgit
(86) International application number: PCT/EP2022/061004
(87) International publication number: WO 2022/229155

(56) References cited:
- WENPING LI ET AL: "Radiosynthesis of the HIV integrase inhibitor [18F]MK-0518 (Isentress)", JOURNAL OF LABELLED COMPOUNDS AND RADIOPHARMACEUTICALS, vol. 53, no. 7, 11 May 2010 (2010-05-11), GB, pages 517 - 520, XP055166071, ISSN: 0362-4803, DOI: 10.1002/jlcr.1778
- LI XIAOMIN ET AL: "Method Development and Validation for Determination ofp-Toluenesulfonoxypropyl-(+)-Dihydrotetrabenazine Enantiomeric Purity by HPLC on a Chiral Stationary Phase", CHROMATOGRAPHIA, VIEWEG UND TEUBNER VERLAG, DE, vol. 80, no. 3, 30 January 2017 (2017-01-30), pages 483 - 488, XP036176118, ISSN: 0009-5893, [retrieved on 20170130], DOI: 10.1007/S10337-017-3255-9
- ORY DIETER ET AL: "Retention of [18F]fluoride on reversed phase HPLC columns", JOURNAL OF PHARMACEUTICAL AND BIOMEDICAL ANALYSIS, ELSEVIER B.V, AMSTERDAM, NL, vol. 111, 9 April 2015 (2015-04-09), pages 209 - 214, XP029171539, ISSN: 0731-7085, DOI: 10.1016/J.JPBA.2015.04.009

## Description

### Field of the Invention

The present invention generally relates to an improved method of analysing radiochemical purity and chemical impurities in a radiopharmaceutical composition, using reversed phase HPLC.

### Background

Radiopharmaceuticals are compounds labelled with a radioactive element, suitable for *in vivo* mammalian administration, for use in the field of medical imaging, diagnosis or therapy. Radiopharmaceutical compositions comprise a radio-labelled compound or a pharmaceutically acceptable salt thereof, solvent, and one or more stabilizers. Before a radiopharmaceutical composition, or 'drug product' can be administered to a patient, it must undergo a thorough quality control (QC) process. Quality control or quality assurance standards are published by USP (usp.org), for example, General Chapter <825> Radiopharmaceuticals - Preparation, Compounding, Dispensing, and Repackaging, originally published 1 June 2019 and General Chapter <1823> Positron Emission Tomography Drugs - Information. One of the key tests in the QC process is quantifying chemical impurities and radiochemical purity (RCP) of the drug product. This analysis is routinely carried out by high pressure liquid chromatography (HPLC).

Radiopharmaceuticals are manufactured within a short time frame prior to administration to a patient. This is because the radioactivity of the radioactive element in the radiopharmaceutical decreases over time due to radioactive decay, thus leading to a decrease in the radioactive potency of the product. The QC process must be carried out on a radiopharmaceutical composition and all QC tests must be passed prior to it being released for administration to a patient. It is therefore desirable for the time needed to carry out the QC process to be as short as possible.

Fluorine-18 ([¹⁸F]) is a radioactive fluorine isotope commonly used in radiopharmaceuticals suitable for use in diagnosis. Fluorine-18 decay occurs by positron emission (97%) and electron capture (3%). As the radioisotope [¹⁸F] decays, the positrons emitted are utilised in positron emission tomography (PET) imaging. This *in vivo* imaging method is used, *inter alia,* in cardiac imaging, tumour imaging and brain imaging. The half-life of [¹⁸F] is 109.77 minutes. That is, the amount of radioactivity of a radiopharmaceutical sample containing [¹⁸F] halves every 109 minutes. Accordingly, the speed with which a radiopharmaceutical drug product sample can be analysed and released for administration to a patient is critical.

The HPLC analysis of [¹⁸F] radiopharmaceuticals requires the analysis of both chemical impurities and radio-impurities in the presence of high concentrations of drug product matrix components. The radio impurities all include the [¹⁸F] radionuclide. HPLC analysis of [¹⁸F] radiopharmaceuticals can be further complicated by the nature of the [¹⁸F] radionuclide. In the radiopharmaceutical, the [¹⁸F] nuclide is often incorporated into a hydrophobic compound. However, the free [¹⁸F]fluoride that may be present and must be quantified in the drug product prior to being released for administration to a patient is very hydrophilic. The analysis is further complicated by the adsorption of free [¹⁸F]fluoride to the stationary phases used in the reversed phase HPLC analysis of more hydrophobic PET radiopharmaceuticals, making quantitation of free [¹⁸F]fluoride problematic. Ensuring elution of free [¹⁸F]fluoride from a reversed phase HPLC column as a quantifiable peak has traditionally required a mobile phase with the pH controlled at a near-neutral pH. However, not all analysis of the more hydrophobic PET radiopharmaceutical is best achieved at a neutral pH. A complex solvent system is needed to provide the mobile phase in the HPLC system. Some HPLC analyses of radiopharmaceuticals are best achieved in an acidic mobile phase. However, free [¹⁸F]fluoride cannot be quantified under acidic conditions. Wenping Li et al., J. Label Compd. Radiopharm 2010, 53, 517-520, discloses the purification of [¹⁸F]MK-0518 on a reverse-phase HPLC system.

It would be of great advantage to be able to develop an HPLC separation method without any constraint imposed by the needs of free [¹⁸F]fluoride. The present invention allows this to be achieved, providing a simplified process that enables rapid analysis of free [¹⁸F]fluoride and chemical impurities in a radiopharmaceutical drug product.

### Summary of the Invention

The present invention relates to an improved analytical HPLC method for [¹⁸F]fluoride quantification and chemical impurity quantification in a [¹⁸F] radiopharmaceutical product. An aspect of the invention relates to a method of analysing radiochemical purity and chemical impurities in a radiopharmaceutical composition, using reversed-phase HPLC, wherein the radiopharmaceutical composition comprises a compound or a pharmaceutically acceptable salt thereof, labelled with [¹⁸F], and wherein the method comprises
(a) providing a sample of the radiopharmaceutical composition to the HPLC column while the mobile phase is water, or water and an organic solvent, or is a neutral pH buffer; or a neutral pH buffer and an organic solvent and subsequently
(b) providing a different mobile phase comprising an acid modifier, water and organic solvent to the HPLC column.

This method allows a sample comprising a radiopharmaceutical composition to be analysed by reversed-phase HPLC wherein free [¹⁸F]fluoride is eluted during step (a), separate from the remaining components of the sample, including the radiopharmaceutical compound or salt thereof that is labelled with [¹⁸F], which are eluted during step (b).

When the sample of the radiopharmaceutical composition (or 'drug product') is provided to the HPLC column in step (a), the mobile phase ('mobile phase A') contains only organic solvent and water, or organic solvent and neutral pH buffer. The radiopharmaceutical composition itself initially provides a local pH controlled environment within mobile phase A. That is, when step (a) is carried out using water and organic solvent, then the sample plug provides the pH control for fluoride recovery. When step (a) is carried out using neutral buffer and organic solvent, then the pH control is provided by the mobile phase. This enables the recovery of free [¹⁸F]fluoride, as the mobile phase provided by the drug product matrix ensures a pH environment (pH 5 to 7) favourable to the elution of the free [¹⁸F]fluoride as an easily quantifiable chromatographic peak.

In step (b), after the free [¹⁸F]fluoride has eluted down the column or begun eluting down the column, a different mobile phase ('mobile phase B'), comprising water, organic solvent and an acid modifier is partitioned into the column. The acid modifier causes mobile phase B to have an acidic pH. The acidic pH of mobile phase B confers an advantage for the controlled elution of drug product matrix components and can provide a more optimum mobile phase pH for the elution through the column of the remaining components of the sample. The method of the invention allows for rapid and accurate quantification of [¹⁸F]fluoride and good resolution of chemical impurities and radio-impurities.

The invention gives the freedom to choose any mobile phase that gives the best possible analysis of the radiopharmaceutical composition, without the method being constrained by the needs for quantitation of free[¹⁸F]fluoride. Optimised separation of radio-impurities and drug-related chemical impurities is often achieved with an acidic mobile phase.

### Figures

Figure 1 shows a comparison between HPLC chromatograms of the method of the invention and current HPLC solvent systems that are designed specifically to enable quantification of free [¹⁸F]fluoride.
Figure 2 shows a comparison between the resolution of radio-impurities in HPLC chromatograms of a sample of a radiopharmaceutical composition labelled with [¹⁸F]fluoride according to a current method and according to the method of the invention.
Figure 3 shows a comparison between the resolution of radio-impurities in HPLC chromatograms according to a current method and the method of the invention based on gradients in the mobile phase system.
Figure 4 shows a comparison between the resolution of chemical impurities in HPLC chromatograms according to a current method and the method of the invention based on the mobile phase system.
Figure 5 shows HPLC traces according to the method of the invention and gradients used in the mobile phases to separate chemical impurities and radio-impurities.
Figure 6 illustrates solvent concentration gradients used in the mobile phases in a method of the invention.

### Detailed Description of the Invention

Radiopharmaceuticals and radiopharmaceutical compositions are typically prepared at radio-pharmacies or PET Centres, where they also undergo quality control testing. Once a drug product has passed the quality control (QC) process and has been released for administration to a patient, it may be administered to a patient on site, where PET scanning facilities exist, or it may be shipped to a PET scanning facility.

One of the key tests in the QC process is quantifying chemical impurities and the radiochemical purity (RCP) of the drug product. This analysis is routinely carried out by high pressure liquid chromatography (HPLC). HPLC achieves the separation of the various components of a liquid composition due to the different interactions of the components with the stationary phase (usually a silica-based column) and the mobile phase, or eluent, that passes through the column. The chemical nature of the components determines their affinity for the stationary phase, based on the intermolecular interactions, and the time spent on the column before eluting. The mobile phase composition may be constant (i.e. isocratic elution) or may be changed (i.e. gradient elution) during the separation process. In order to separate compositions comprising components that have very different chemical natures, additional solvents may be introduced to the column, typically by increasing the proportion of solvent in the mobile phase. A detection unit (e.g. a UV detector) and computer processing unit record the time analytes elute and generate a chromatogram showing the signal peaks for the separated components. The signal peaks provide qualitative and quantitative data and allow for the calculation of the concentration of a component. The retention time (t_{R}) refers to the time between injection of a component into the system and its elution off the column (detection). Reversed-phase HPLC describes the chromatography mode that uses a polar mobile phase and a non-polar stationary phase. Compounds are selectively eluted from the stationary phase as the mobile phase is made increasingly non-polar, usually by the addition of increasing levels of organic solvent, that is, by increasing the proportion of organic solvent in the mobile phase. Polymer-based columns can be used for reverse phase chromatography. However, the most common stationary phases are prepared by the covalent bonding of an organic material to a silica particle. The organic material can be a phenyl type compound, or more usually an alkyl chain. The alkyl chain length determines the hydrophobicity of the stationary phase and HPLC columns are available with alkyl chain lengths ranging between C2 and C30. A common stationary phase is C18 bonded to silica, and typical mobile phases include an aqueous mixture of water and organic solvent, for example acetonitrile or methanol.

Radiochemical purity (RCP) can be determined using HPLC and can be defined as the ratio of the (radio-labelled) drug substance peak to the total (radio-labelled) peaks in the chromatogram.

The term radiopharmaceutical has its conventional meaning and refers to a radioactive compound suitable for *in vivo* mammalian administration for use in diagnosis or therapy. Any radiopharmaceutical composition comprising an [¹⁸F]-labelled radiopharmaceutical and pharmaceutically acceptable excipients, stabilisers, solvents and the like, suitable for injection into a mammal, for example a human, can be analysed by the HPLC method of the present invention.

The term "comprising" has its conventional meaning throughout this application and implies that the composition must have the components listed, but that other, unspecified compounds or species may be present in addition.

### Radiopharmaceutical compositions

The following description serves to exemplify various possible components of radiopharmaceutical compositions. Radiopharmaceutical compositions for use in the method of the present invention comprise a radiopharmaceutical, a solvent, a solubiliser and a radiostabilizer. The radiopharmaceutical composition for use in the method of the present invention may comprise a [¹⁸F]-labelled radiopharmaceutical that is analysable by reversed-phase HPLC. The radio-labelled compound may comprise a [¹⁸F]-labelled radiopharmaceutical, or a pharmaceutically acceptable salt thereof. Examples of such [¹⁸F]-labelled radiopharmaceuticals include [¹⁸F]FMAU (2'-deoxy-2'-[¹⁸F]fluoro-5-methyl-1-beta-D-arabinofuranosyluracil), [¹⁸F]FMISO (F18 Fluoromisonidazole), [¹⁸F]FHBG (9-(4-18F-Fluoro-3-[hydroxymethyl]butyl)guanine), [¹⁸F]AV-45, [¹⁸F]AV-19, [¹⁸F]AV-1, [¹⁸F] Flutemetamol, [¹⁸F]FES (Fluoroestradiol F18), [¹⁸F] Flurpiridaz, [¹⁸F]K5, [¹⁸F]HX4, [¹⁸F]W372, [¹⁸F]VM4-037, [¹⁸F]CP18, [¹⁸F]ML-10, [¹⁸F]T808, [¹⁸F]T807, 2-[¹⁸F]fluoromethyl-L-phenylalanine, or combinations thereof.

The radio-labelled compound may be a compound of Formula (I): 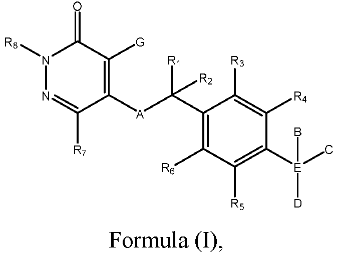
wherein A is selected from N(R⁷), S, O, C(=O), C(=O)O, NHCH₂CH₂O, a bond, or C(=O)N(R⁷);
when present, B is selected from hydrogen, alkoxyalkyl, alkyloxy, aryl, C₁-C₆ alkyl optionally substituted with an imaging moiety, heteroaryl, and an imaging moiety;
when present, C is selected from hydrogen, alkoxyalkyl, alkyloxy, aryl, C₁-C₆ alkyl optionally substituted with an imaging moiety, heteroaryl, and an imaging moiety;
D is selected from hydrogen, alkoxyalkyl, alkyloxy, aryl, C₁-C₆ alkyl optionally substituted with an imaging moiety, heteroaryl, and an imaging moiety; or
C and D, together with the atom to which they are attached, form a three- or four-membered carbocyclic ring;
G is halo or haloalkyl;
n is 0, 1, 2, or 3;
R¹, R², R³, R⁴, R⁵, R⁶ and R⁷ are independently selected from hydrogen, C₁-C₆ alkyl optionally substituted with an imaging moiety, and an imaging moiety;
R⁸ is C₁-C₆ alkyl, optionally substituted with an imaging moiety; and
E is selected from a bond, carbon, and oxygen, provided that when E is a bond, B and C are absent and D is selected from aryl and heteroaryl, and provided that when E is oxygen, B and C are absent and D is selected from hydrogen, alkoxyalkyl, aryl, C₁-C₆ alkyl optionally substituted with an imaging moiety, and heteroaryl;
provided that at least one imaging moiety is present in Formula (I).

Substituent A of Formula (I) may be O. R⁸ may be tert-butyl. G may be chloro. The imaging moiety may be any radio-isotope as referenced herein, for example ¹⁸F.

The radio-labelled compound may comprise [¹⁸F]flurpiridaz (also known as Flurpiridaz 18F), which has the following structure:

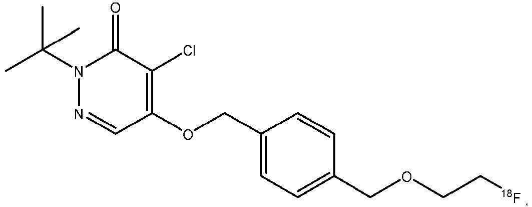

The radio-labelled compound may be a compound selected from: 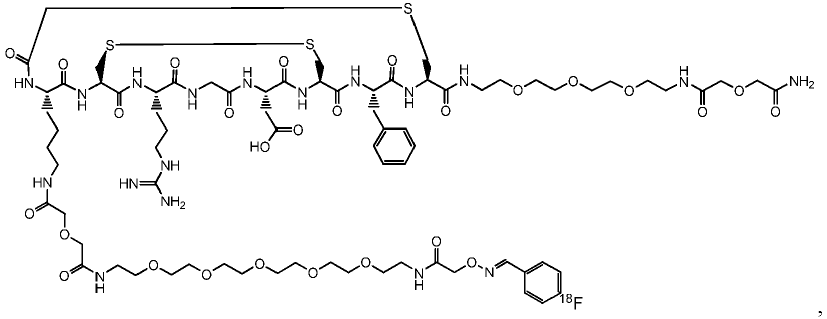 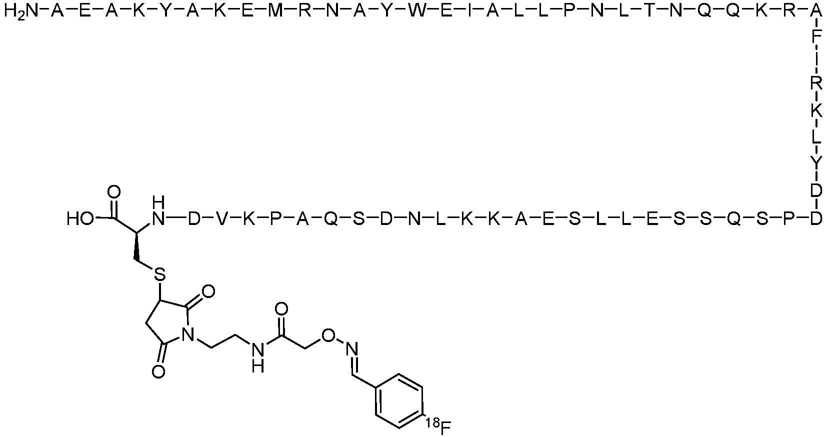 or

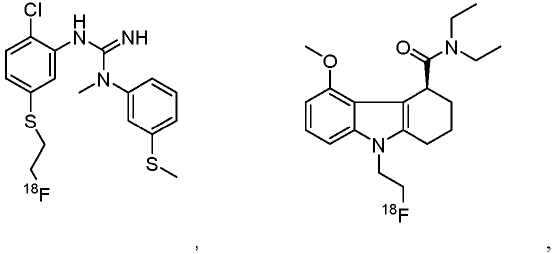

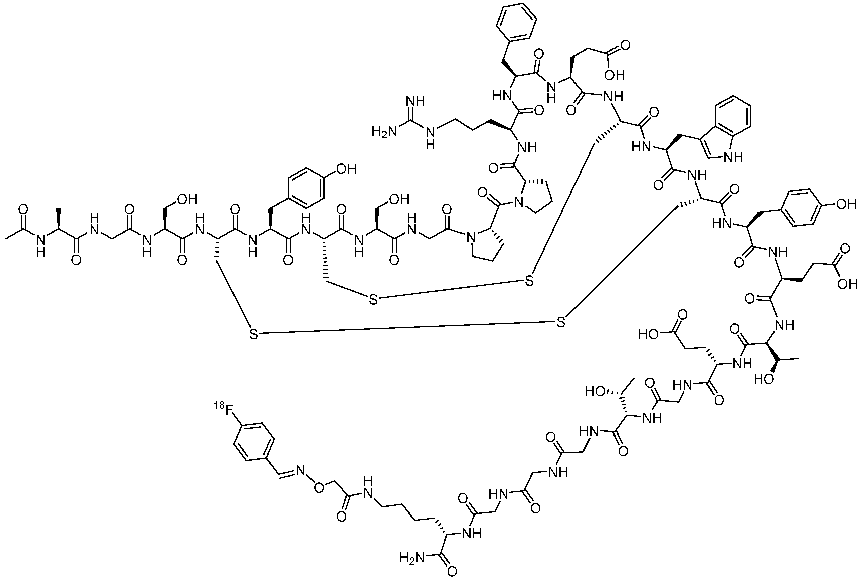

The radiopharmaceutical composition may contain additional optional excipients. For example, such additional optional excipients include: an antimicrobial preservative, pH-adjusting agent (buffer), filler, solubiliser or osmolality adjusting agent. Stabilizers are needed in radiopharmaceutical preparations to reduce the formation of radioimpurities during their shelf life. Suitable stabilizers include ethanol, sodium ascorbate, ascorbic acid, maleic acid, *para* amino benzoic acid, polysorbates, polyethylene glycols, gentisic acid and calcium chloride, among others.

The radiopharmaceutical composition may comprise a biocompatible carrier. The biocompatible carrier is a fluid, especially a liquid, in which the radiopharmaceutical can be suspended or preferably dissolved, such that the composition is physiologically tolerable, i.e. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is isotonic); an aqueous buffer solution comprising a biocompatible buffering agent (e.g. phosphate buffer); an aqueous solution of one or more tonicity-adjusting substances (e.g. salts of plasma cations with biocompatible counterions), sugars (e.g. glucose or sucrose), sugar alcohols (e.g. sorbitol or mannitol), glycols (e.g. glycerol), or other non-ionic polyol materials (e.g. polyethyleneglycols, propylene glycols and the like). Preferably, the biocompatible carrier is pyrogen-free water for injection, isotonic saline or phosphate buffer.

A radiopharmaceutical composition may, for example, comprise a radio-labelled compound, ascorbic acid, ethanol and cyclodextrin. An exemplary radiopharmaceutical composition comprises ascorbic acid and ethanol, cyclodextrin, for example hydroxypropyl-betacyclodextrin (HPbCD), and the radio-labelled compound [¹⁸F]flurpiridaz. The ascorbic acid can be present in an amount from about 1 to about 100 mg/mL, the ethanol can be present in an amount from about 2 to about 10% (v/v) and the HPbCD can be present in an amount of from about 1 to about 1000 mg/mL, for example from about 1 to about 350 mg/mL, for example from about 1 to about 100 mg/mL, for example from about 30 mg/mL to about 70 mg/mL, for example about 50 mg/mL. Ethanol typically makes up less than or equal to 10% by volume of the composition.

### HPLC Method

[¹⁸F]Fluoride HPLC analysis is generally challenging as many factors need to be considered. In an acidic environment the free [¹⁸F]fluoride absorbs onto the stationary phase of the column and elution is difficult: the free [¹⁸F]fluoride does not elute as a discrete chromatographic peak that can be quantified.

The present invention relates to a method of analysing radiochemical purity and chemical impurities in a sample comprising a radiopharmaceutical composition, using reversed phase HPLC, wherein the radiopharmaceutical composition comprises a compound or a pharmaceutically acceptable salt thereof, labelled with [¹⁸F], and (a) providing the sample comprising the radiopharmaceutical composition to the HPLC column while the mobile phase is water, or water and an organic solvent, or a neutral pH buffer, or a neutral pH buffer and an organic solvent; and subsequently (b) providing a different mobile phase comprising an acid modifier, water and organic solvent to the HPLC column.

Typically, the radiopharmaceutical composition comprises water, ethanol, a buffer or stabiliser, such as ascorbic acid, and cyclodextrin.

When the sample of the radiopharmaceutical composition (the drug product) is provided to the HPLC column, the mobile phase ('mobile phase A') contains only organic solvent and water. Alternatively, mobile phase A may comprise a neutral pH buffer, or a neutral pH buffer and an organic solvent.

In the case where 'mobile phase A' contains only organic solvent and water, this mobile phase provides no pH control. Once the sample of the radiopharmaceutical composition is injected into the HPLC column, the matrix of the sample establishes the pH control. That is, the matrix of the sample provides a local micro-environment around the fluoride ion (the free [¹⁸F]fluoride) within the initial mobile phase ('mobile phase A'), which ensures the fluoride elutes down the column. Free [¹⁸F]fluoride elutes down the column, because the injected sample by virtue of its matrix components, provides a pH environment between pH 5 and 7. This creates a favourable environment for elution of free [¹⁸F]fluoride off the column and results in an easily quantifiable chromatographic peak.

The 'matrix' of the drug product is defined as every component present in the drug product (for example the radiopharmaceutical composition) other than the chemical compound radio-labelled with [¹⁸F]fluoride. The matrix may for example comprise ethanol, buffer salts (formate, phosphate, citrate, acetate, and/or one or more amino acids), radiostabilisers, solubilisers, as set out above for the radiopharmaceutical composition.

After the free [¹⁸F]fluoride has eluted down the column or has begun eluting down the column, a different mobile phase ('mobile phase B') is partitioned into the column. Mobile phase B comprises an acid modifier, water and organic solvent. Any acid modifier can be used in mobile phase B and imbues mobile phase B with an acidic pH. An acidic pH is defined to be less than 7.0. Preferably the pH of mobile phase B is less than 5.0, more preferable less than 4.5, between 0 and 5.0, between 0 and 4.0, between 1.0 and 3.0, between 1.5 and 2.5, for example about 2.0 Examples of suitable acid modifiers in the mobile phase include, among others, trifluoroacetic acid (TFA), formic acid, acetic acid, phosphoric acid, citric acid and acidic phosphate buffers. The proportion of the acid modifier in mobile phase B may be constant or increased gradually. Where a mixture of different components is used in mobile phase B, their respective proportions may be increased or reduced on a gradient.

A pH modifier is any substance added to the mobile phase that assists in controlling or adjusting the pH. An acid modifier is any substance added to the mobile phase that establishes an acidic pH, usually defined as any pH less than 7.0. For the purposes of the present invention, the acid modifier is defined as any substance added to the mobile phase that establishes an acidic pH of 5.0 or below. A basic modifier is any substance added to the mobile phase that establishes a basic pH, usually defined as any pH greater than 7.0, on a polymer-based column.

Generally, trifluoroacetic acid (TFA) is a very useful pH modifier (acid modifier) in the mobile phase, or eluent, in HPLC, as it assists in good separation of components of a sample, including chemical and radiochemical impurities. However, HPLC methods for analysis of [¹⁸F] radiopharmaceuticals (for example in the quality control testing of PET products radiolabelled with [¹⁸F]) do not usually use TFA as a mobile phase acid modifier, because TFA does not allow for the quantification of free [¹⁸F]fluoride. The inventors have found that by the radiopharmaceutical composition sample plug itself providing the initial, temporary, pH control of mobile phase A (which does not contain any pH modifier), simple analysis of the free [¹⁸F] fluoride present in the sample is achieved.

Subsequently, the mobile phase can be adjusted to optimise the separation of the remaining components of the sample. An acid modifier is only partitioned into the mobile phase in step (b) (as a component of 'mobile phase B'), once the sample (in particular the free [¹⁸F]fluoride) has started eluting down the column. The acid modifier can be partitioned into the mobile phase in an increasing gradient, giving precise pH control, which contributes to the control of elution of the remaining components off the column.

Unlike the free [¹⁸F]fluoride, which is hydrophilic, the remaining components are more hydrophobic and the mobile phase needs to become increasingly non-polar in order for these components to elute through the column. This can be achieved, for example, by increasing the proportion of organic solvent relative to water in the mobile phase, to cause the more hydrophobic components to elute. The mobile phase is supplied to the column from two or more solvent reservoirs. By changing the ratio of the contributions from the reservoirs, for example from a first and a second reservoir, to the column, the composition of the mobile phase can be altered. The HPLC apparatus may comprise at least two reservoirs, for example, two, three or four reservoirs. Reservoirs may, for example, contain different components or different concentrations of certain components. The composition of each reservoir remains constant, but the composition of a mobile phase may be altered by changing the ratio of contribution from each reservoir to the mobile phase at any given time.

Mobile phase A is defined herein as the mobile phase running through the column, which does not contain any acid modifier. Mobile phase B is defined herein as the mobile phase running through the column that comprises an amount of acid modifier. The composition of mobile phase B may be changed, by altering the proportions of the components, e.g. on a gradient, by altering the contribution from the reservoirs.

Mobile phase A is water and an amount of organic solvent. For example, mobile phase A may contain 0-60% organic solvent, for example 10-60% organic solvent, for example 30% organic solvent to 50% organic solvent, in water. Mobile phase A may comprise one or more organic solvents.

In an alternative embodiment of the invention, one or more neutral pH buffers can be used as mobile phase A. A suitable neutral pH buffer is any buffer that maintains the pH in the range of approx. 4.0 to 8.0. Suitable buffers include, but are not limited to, phosphate, ammonium acetate, ammonium formate, sodium acetate, tartrate, citrate-phosphate, citrate, pyridine-formate, pyridine-acetate, dimethylglutarate, succinate, phthalate, piccoline acetate, cacodylate buffer, tris-maleate, sodium hydrogen maleate buffer, sodium arsenate, and imidazole. Preferred neutral pH buffers include phosphate, ammonium acetate, and ammonium formate. A preferred concentration range for the neutral pH buffer is 0.1 to 100 mM.

In mobile phase B, an acid modifier is introduced to the column together with organic solvent and water. The proportion of organic solvent in mobile phase B may be between 1% and 100% organic solvent, for example, 30% and 100% organic solvent. Where the proportion of organic solvent is less than 100%, the balance is water. The amount of organic solvent in mobile phase B may be increased on a gradient, for example, from 1% to 95%, from 1 % to 50%, from 30% to 95%, from 30% to 80%, from 50% to 80% or from 50% to 70% organic solvent, in water, in order to assist elution from the column. Mobile phase B may comprise one or more organic solvents.

The one or more organic solvents may be the same or different in mobile phase A and mobile phase B. Preferably, the one or more organic solvents are the same in mobile phase A and mobile phase B. The proportion of acid modifier may be 0.05% to 1%, for example 0.1% to 0.25%. The proportion of acid modifier may remain constant, for example, at 0.1%, or may increase proportionally to the increase of the proportion of the solvent it is in. This may depend on the HPLC apparatus used.

The organic solvent in mobile phase A and/or mobile phase B may, for example, be selected from acetonitrile or methanol. Mobile phase A may, for example, initially comprise 30% MeCN and 70% water. The proportion of MeCN in the mobile phase running through the column may be increased on a gradient, by adjusting the contribution from the solvent reservoirs. Mobile phase B may comprise MeCN, water and an acid modifier. The steepness of the gradient (the increase of the proportion of organic solvent over time) will depend on the properties of the sample components. The final proportions of the mobile phase running through the column may, for example, be 70% MeCN, up to 29.9% water and at least 0.1% acid modifier. In another example, mobile phase A and B may comprise MeOH and water. For example, mobile phase A may comprise 50% MeOH and 50% water. The proportion of MeOH in mobile phase B may be increased on a gradient, by adjusting the contribution from the solvent reservoirs. Mobile phase B additionally comprises an acid modifier. The acid modifier may, for example, be trifluoroacetic acid, formic acid acetic acid, phosphoric acid, citric acid and acidic phosphate buffers or a phosphate buffer.

Control of pH is considered crucial in chromatography. This is because ionisable functional groups behave differently in reversed phase chromatography depending on their ionised state. The charge of the ionisable group will change depending on the pH. Therefore, when carrying out analysis, such as HPLC, control of the pH is often very important. Control of pH can be achieved by pH modifiers, for example, acid modifier to achieve an acidic pH (and base modifiers to achieve a basic pH on polymer-based columns). Buffers can also be used to achieve robust pH control and balance out fluctuations.

Contrary to standard procedures in the art, for an analysis of an analyte (e.g. fluoride) that requires pH control, there is no pH modifier comprised in mobile phase A. Rather, the pH control (about pH 5 to pH 7) in the vicinity of the fluoride arises from the drug product matrix itself. This enables the elution and accurate quantification of free [¹⁸F]fluoride. When mobile phase A is a neutral buffer then the pH control is provided by the neutral buffer. The subsequent introduction of an acid modifier in mobile phase B provides an acidic environment and allows for the good separation and quantification of the remaining components of the sample, including chemical impurities, without interfering with the elution and quantification of free [¹⁸F]fluoride. The method of the invention allows for the easy quantification of [¹⁸F]fluoride as well as good resolution of chemical impurities in the sample. As is described in further detail below, the addition of the acid modifier is very important for control of the large peak in the chromatogram due to the matrix materials of a drug product.

The time period between the introduction of the drug product sample to the column and the introduction of mobile phase B to the column can be as little as 1 second. Preferably, the time period between the introduction of the drug product sample to the column and the introduction of mobile phase B to the column is between 1 second and 5 minutes, preferably between 5 seconds and 60 seconds, more preferably between 5 seconds and 30 seconds, for example about 20 seconds. In another embodiment, the time period between the introduction of the drug product sample to the column and the introduction of mobile phase B to the column is zero seconds.

Another advantage of the method of the invention is that the HPLC analysis of the drug product sample can be carried out within approximately 10 minutes. This is in contrast to current methods, which usually take approximately 20 minutes. As has been explained, the radiopharmaceutical composition is both prepared and subjected to quality control testing within a short timeframe prior to administration to a patient, in view of the radioactive decay of the radionuclide. Halving the time required to carry out the HPLC analysis is hugely advantageous. The method of the invention enables the later separation of the non-free [¹⁸F] fluoride-components to be carried out in the most optimum way possible, thus allowing for greatly reduced analysis times. This is further explained with reference to the Figures.

The HPLC method of the present invention is applicable to any reversed phase HPLC analysis for radiopharmaceutical drug products radiolabelled with [¹⁸F]fluoride. Any silica-based reversed phase HPLC column (C2 to C30) of any column dimension, particle size and pore size is suitable for use in the method of the invention. Exemplary columns that can be used in the present invention include Zorbax SB-C18 P/N 822975-902 (*Agilent, USA*), and Kinetex C18, P/N 00B-4462-E0 (*Phenomenex Inc*.).

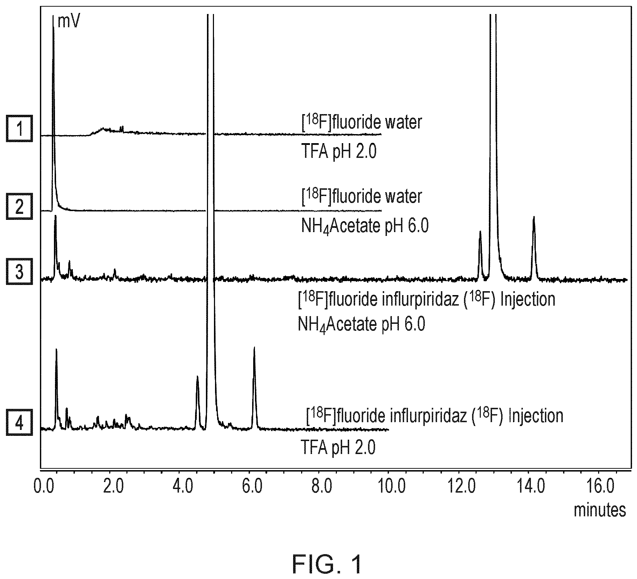

Figure 1 depicts an HPLC chromatogram. The traces are numbered from top to bottom as trace 1, trace 2, trace 3 and trace 4.

Trace 1 depicts the chromatogram generated by carrying out HPLC on a sample of free [¹⁸F]fluoride in water. The mobile phase initially does not contain acid. TFA is added to the mobile phase after sample injection. Consequently, the pH is lowered to approximately 2.0. However, as can be seen from trace 1, when in a sample matrix of just water, the free [¹⁸F]fluoride elutes from the column as a wide peak between about 2.3 minutes and 3.8 minutes. The wide peak is not quantifiable.

Trace 2 depicts the chromatogram generated by carrying out HPLC on a sample of free [¹⁸F]fluoride in water. The mobile phase includes buffer salt ammonium acetate (NH₄ acetate) at pH 6.0. The free [¹⁸F]fluoride elutes easily and gives a quantifiable peak in the chromatogram. However, this mobile phase is more difficult for radio-pharmacies or PET Centres to handle. The use of buffered mobile phases imposes an additional burden on radio-pharmacies performing the analysis prior to release of the drug product for administration to a patient. Preparation of mobile phases with buffer salts and pH modification is more labour intensive and instruments must be rigorously washed after using buffered mobile phases in order to avoid precipitation of the buffer salts within the instrument.

Trace 3 depicts the chromatogram generated by carrying out HPLC on a sample of the radiopharmaceutical drug product labelled with [¹⁸F]fluoride, according to a current standard method, using a C18 column. The drug product comprises [¹⁸F ]flurpiridaz (GE Healthcare, USA), ascorbic acid, ethanol and cyclodextrin (HP-b-cyclodextrin, Cyclodextrin HPB EMPROVE, Merck P/N 1.42020). The mobile phase includes buffer salt ammonium acetate (NH₄ acetate) at pH 6.0. The free [¹⁸F]fluoride elutes at around 0.5 minutes. The radiopharmaceutical elutes as a large, sharp peak at around 13 minutes, with radio-impurities eluting between 12.5 and 17.5 minutes (small peaks). The chromatogram shows sharp peaks, however, because the mobile phase includes ammonium acetate, the method requires a 20 minute run time. The reason why the method takes 10 minutes longer is because the method is a multi-dimensional analysis. There are the radio-impurities (which are all labelled with [¹⁸F]fluoride) and also chemical impurities, which are not radiolabelled. The product matrix, for example comprising large amounts of ascorbic acid and cyclodextrin, gives a huge response in the HPLC analysis (with detection using absorbance of UV-light). An acidic mobile phase enables these matrix components to elute off the column quickly. However, at a neutral pH, as provided by ammonium acetate, the matrix components take about 10 minutes to elute off the column creating huge peaks in the chromatogram.

Trace 4 depicts the chromatogram generated by carrying out HPLC on a sample of a drug product labelled with [¹⁸F]fluoride, according to the invention. The drug product comprises [¹⁸F]flurpiridaz (*GE Healthcare, USA*), ascorbic acid, ethanol and cyclodextrin. The sample is of the same drug product labelled with [¹⁸F]fluoride as analysed in trace 4. The drug product sample is initially injected onto the C18 column, itself providing a transient mobile phase A. The pH in the sample plug is between 5.0 to 7.0 and is determined by the pH of the drug product. This enables the free [¹⁸F]fluoride to elute quickly at around 1.0 to 1.4 minutes as a sharp, quantifiable peak. Subsequent to the injection of the drug product sample, TFA is partitioned into the column as a component of mobile phase B. The pH is thereby lowered to approximately pH 2.0. The radiopharmaceutical elutes as a strong, sharp peak at around 5 to 6.3 minutes, with radio-impurities eluting between 5.0 and 7.5 minutes (small peaks). The total run time is approximately 10 minutes.

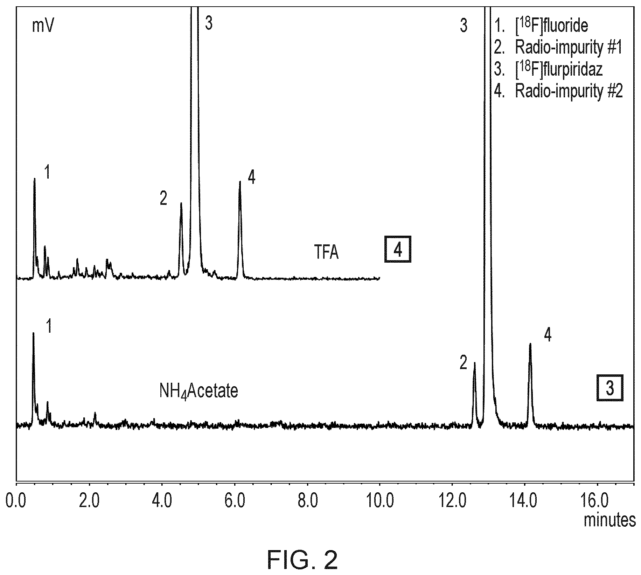

Figure 2 compares trace 3 and trace 4 of Figure 1. The HPLC analysis is carried out on a sample of the same radiopharmaceutical drug product labelled with [¹⁸F]fluoride. In trace 4, the drug product sample is injected onto the column and itself provides a transient pH control in mobile phase A. The free [¹⁸F]fluoride elutes quickly at around 1.0 to 1.4 minutes as a sharp, quantifiable peak (peak 1). Subsequent to the injection of the drug product sample, TFA is partitioned onto the column as a component of mobile phase B. The pH level is thereby lowered to 2.0. The radiopharmaceutical elutes as a sharp peak at around 4.5 to 5 minutes (peak 3), with additional radio- impurities eluting between 4.0 and 6.5 minutes (peaks 2 and 4). The total run time is approximately 10 minutes. In contrast, in trace 3, the same drug product sample is injected onto the column with ammonium acetate providing a pH of 6.0 in the mobile phase. While the free [¹⁸F]fluoride elutes quickly in a sharp, quantifiable peak (peak 1), the radiopharmaceutical (peak 3) and chemical impurities (peaks 2 and 4) have a long retention time and run off the column between 12.5 and 14.5 minutes. A detailed look at this figure shows that in TFA an additional radio-impurity is seen in the tail of the main peak. In other words, being able to use TFA gives a better separation of radio-impurities and a more accurate analysis of the radiopharmaceutical. Having the flexibility to choose the optimum mobile phase is a great benefit.

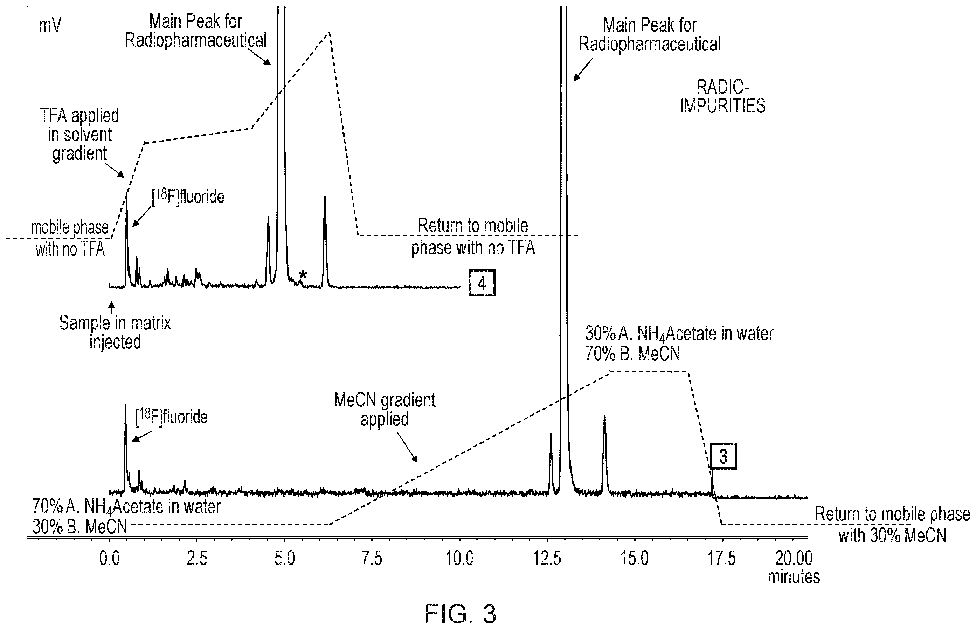

Figure 3 compares the resolution of radio-impurities in trace 3 and trace 4 of Figure 1. Depicted over the chromatograms as a dashed line is the gradient profile of the mobile phase. Reservoir A contains ammonium acetate and water and provides the initial mobile phase. Reservoir B contains 100% MeCN. Contributions from the reservoirs are adjusted to achieve the gradient in the mobile phase in the column. For the analysis in ammonium acetate the dashed line represents the change in the organic solvent content of the mobile phase during the analysis, with the amount increased linearly over the time frame 6.0 to 14.5 minutes. Thereafter, the level of organic solvent is kept constant for a further 1.5 minutes to ensure all compounds elute from the column. At 17.5 minutes the organic solvent is returned to a level suitable for the start of the next analysis.

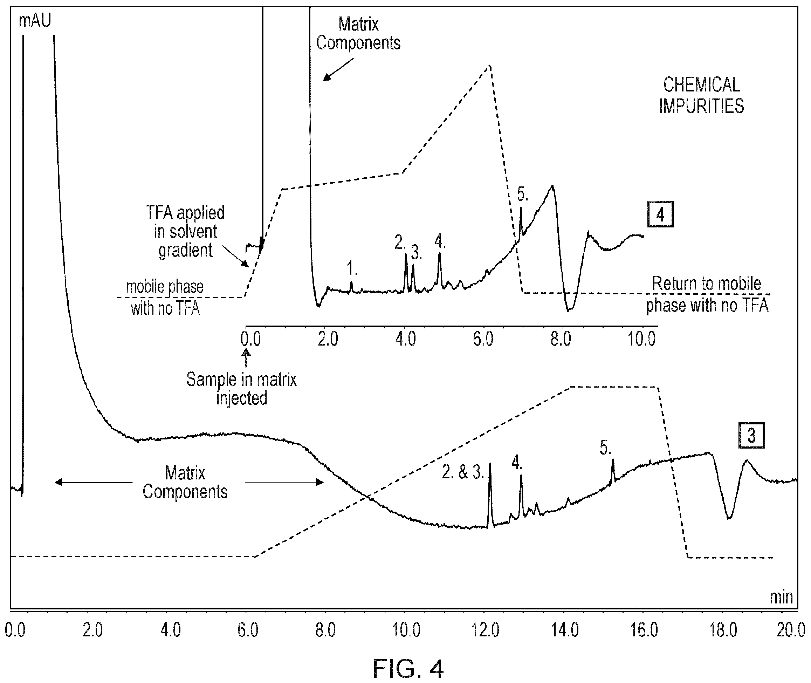

Figure 4 compares the resolution of chemical impurities in trace 3 and trace 4 of Figure 1. Depicted over the chromatograms as a dotted line is the gradient profile of the mobile phase. In trace 4 (mobile phase B including TFA) it can be seen that the matrix components give rise to a large peak in the chromatogram and elute in under 2 minutes. In trace 3 (ammonium acetate), the matrix components (here: ascorbic acid and cyclodextrin) give rise to a large, broad peak, with the elution taking approximately 10 minutes. This is due to the neutral pH of the ammonium acetate buffered mobile phase. It is not possible to analyse any other compounds until the matrix components have fully eluted off the column. Accordingly, the chemical impurity peaks do not elute until between approximately 12 and 16 minutes. It can also be seen that in the trace using TFA in mobile phase B (trace 4), a peak (numbered 1) has been resolved, which cannot be seen in trace 3. Further, in in the trace using TFA in mobile phase B, peaks 2 and 3 are resolved as two separate peaks. In trace 3, peaks 2 and 3 are not separated. This comparison underlines the role played by the acid modifier, for example TFA, in the mobile phase, both in terms of controlling the elution of the matrix materials (giving rise to a sharper peak eluting within under two minutes, compared to a broad peak eluting taking over 10 minutes to elute and affecting the subsequent elution profile), as well as improving the resolution of other components in the sample.

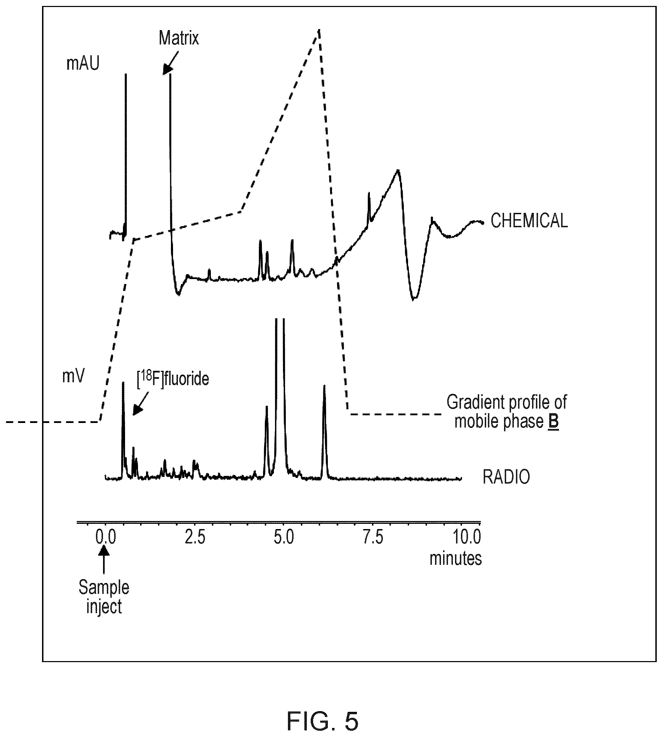

Figure 5 illustrates the multi-dimensional nature of the analysis. Figure 5 depicts two chromatogram traces, showing the resolution of chemical impurities and radiochemical impurities in a sample of an [¹⁸F]-labelled radiopharmaceutical composition (as for trace 4 in Figure 1). The free [¹⁸F]fluoride elutes, giving rise to a well resolved peak allowing for quantification of the amount of free [¹⁸F]fluoride, and the matrix components of the radiopharmaceutical drug product sample elute in a controlled manner. Depicted over the chromatograms is the gradient profile of mobile phase B (dashed line). The gradient is achieved in a binary pump HPLC system - that is, the HPLC system has two solvent reservoirs, referred to here as Reservoir A and Reservoir B. The gradient profile of the mobile phases is set out in Table 1, below. Reservoir A is 30% acetonitrile in water. Reservoir B is 95% v/v acetonitrile, water (5% v/v) and 0.1% v/v TFA.

**Table 1.**

| **Time (min)** | **Mobile phase from Reservoir A** % v/v | **Mobile phase from Reservoir B** % v/v | | **On-column % v/v** | |
|---|---|---|---|---|---|
| | | | | **%MeCN** | **%TFA** |
| 0.0 | 100 | 0 | | 30 | 0.000 |
| 0.5 | 75 | 25 | | 46 | 0.025 |
| 4.0 | 65 | 35 | | 53 | 0.035 |
| 6.5 | 25 | 75 | | 79 | 0.075 |
| 7.0 | 100 | 0 | | 30 | 0.000 |
| 10.0 | 100 | 0 | | 30 | 0.000 |

The gradient means that the concentration of TFA ranges from 0.00 to 0.075% v/v over the course of the analysis (with a gradient profile indicated by the dotted line in Figure 5). But even as the concentration raises from 0.00 to 0.025% in the first part of the gradient there is sufficient acid present to impose pH control (as indicated by the focussing of the peak due to the matrix components).

The solvent gradient described above is carried out on a binary pump HPLC system. However, the invention may also be carried out in a tertiary (three reservoirs) or quaternary (four reservoirs) pump system utilising three or four separate components of the mobile phase from reservoirs A, B, C and D.

Table 2 sets out a mobile phase gradient protocol carried out with 3 eluent channels (i.e. reservoirs A, B and C) for example on a quaternary pump system. Reservoir A is 100% water, Reservoir B is 100% acetonitrile, Reservoir C is 0.15% v/v TFA in water (the amount of TFA can be varied, if required).

**Table 2.**

| **Time (min)** | **Contribution from Reservoir A** % v/v | **Contribution from Reservoir B** % v/v | **Contribution from Reservoir C** % v/v | | **On-column % v/v** | |
|---|---|---|---|---|---|---|
| | | | | | **%MeCN** | **%TFA** |
| 0.0 | 70 | 30 | 0 | | 30 | 0.00 |
| 0.5 | 34 | 46 | 20 | | 46 | 0.03 |
| 4.0 | 27 | 53 | 20 | | 53 | 0.03 |
| 6.5 | 2 | 78 | 20 | | 78 | 0.03 |
| 7.0 | 70 | 30 | 0 | | 30 | 0.00 |
| 10.0 | 70 | 30 | 0 | | 30 | 0.00 |

Where the method of the invention is carried out on an HPLC system with three eluent channels available, the preparation of mobile phases is greatly simplified. In the example set out, the concentration of TFA is maintained constant throughout the region of the chromatogram where compounds that are related to the radiopharmaceutical elute. The concentration of TFA on the column can be increased, if necessary, by adding more TFA when preparing Mobile Phase C.

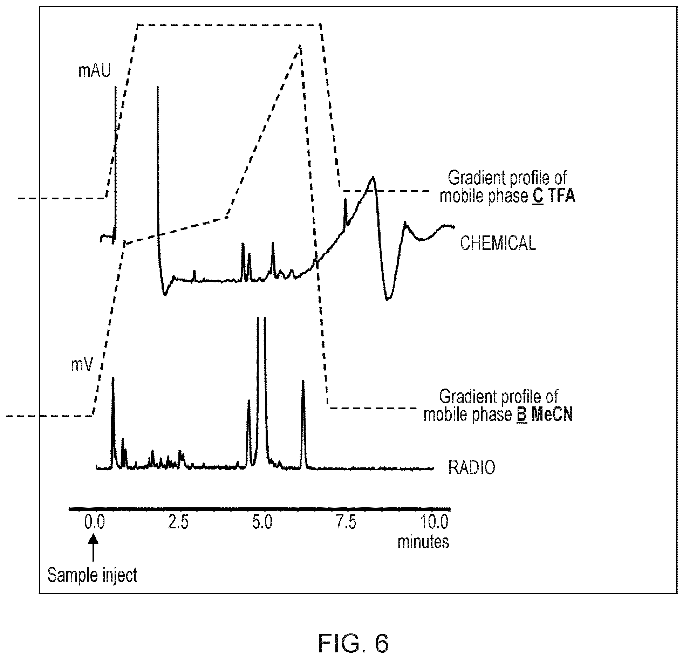

In Figure 6, the gradient profiles of TFA and MeCN are superimposed as dashed lines on a chromatogram that would be expected from the gradients set out in Table 2.

The method of the invention allows for HPLC analysis of free [¹⁸F]fluoride and radio- and chemical impurities, using a simple solvent system. The inventors have found that by using the drug product sample itself to provide a temporary pH control in the vicinity of the fluoride, within mobile phase A, it is possible to achieve quantification of the free [¹⁸F]fluoride and achieve well resolved peaks for the radiopharmaceutical and chemical impurities by virtue of the ability to use an acidic modifier as a component of mobile phase B. The run time of the HPLC analysis according to the method of the invention is less than half compared to current methods, which is a significant advantage.

The invention is described with reference to the following non limiting examples.

### Examples

### Quantification of free [¹⁸F]fluoride and Accuracy of [¹⁸F]fluoride recovery using the method of the invention

The data in Table 4 is a comparison of the HPLC analysis of free [¹⁸F]fluoride in the same radiopharmaceutical composition comprising [¹⁸F]flurpiridaz, using a current method (ammonium acetate) and the method of the invention (TFA), using the composition itself to provide the initial transient pH control in mobile phase A, followed by TFA as an acid modifier in mobile phase B. A known amount of free [¹⁸F]fluoride has been added to the sample in order to demonstrate the accuracy of the determination.

### Level of free [¹⁸F]fluoride present from activity measurement (background corrected) : 0.7%

**Table 4.**

| **Peak #** | **Peak Name** | **Area %** | |
|---|---|---|---|
| | | **Ammonium Acetate ¹⁾** | **Trifluoroacetic acid ²⁾** |
| 1. | [¹⁸F]fluoride | 0.7 | 0.7 |
| 2. | Radio-impurity #1 | 0.8 | 0.8 |
| 3. | [¹⁸F]flurpiridaz | 97.4 | 97.4 |
| 4. | Radio-impurity #2 | 1.2 | 1.1 |

Radio-chromatograms are analysed according to the QC procedure, applying a reporting threshold of 0.3% (i.e. impurities present at a level below 0.3% are not reported)
1) Analysis performed on a Zorbax C18 (50 x 4.6 mm) 1.8 µm column. The mobile phase is at near neutral pH, ammonium acetate (pH 6).
2) Analysis performed on a Kinetex C18 (50 x 4.6 mm) 2.6 µm column. The mobile phase is initially aqueous MeCN (no pH modifier) with a transition to 0.1% TFA during gradient.

Both methods give a relative area of 0.7% for the free [¹⁸F]fluoride peak (peak 1).

The following example and Table 5 relate to a method of incorporating neutral buffer, based on an HPLC system with a quaternary pump system. Table 5 sets out a mobile phase gradient protocol carried out with 4 eluent channels (i.e. reservoirs A, B, C and D) for example on a quaternary pump system. Reservoir A is 30% acetonitrile in water, Reservoir B is 0.10% v/v TFA in 95% acetonitrile (the amount of TFA can be varied, if required), Reservoir C is 10 mM ammonium acetate in water (the amount of ammonium acetate can be varied, if required, or an alternative neutral buffer can be chosen) and Reservoir D is 100% acetonitrile.
Reservoir A: 30% MeCN
Reservoir B: 0.10% TFA in 95% MeCN
Reservoir C: 10 mM ammonium acetate (NH₄Ac) in water
Reservoir D: 100% MeCN

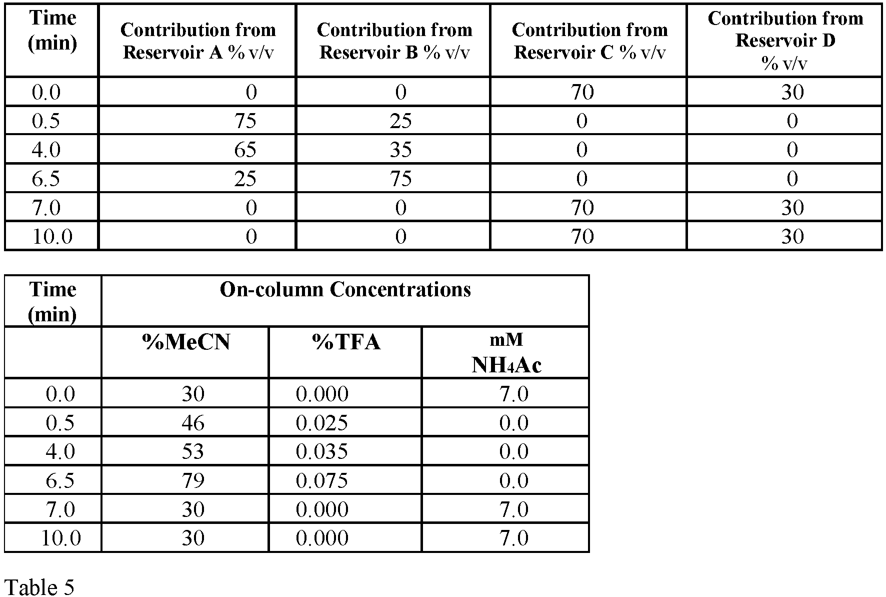

It will be readily understood by those persons skilled in the art that the embodiments of the invention described herein are capable of broad utility and application. Accordingly, while the invention is described herein in detail in relation to the exemplary embodiments, it is to be understood that this disclosure is illustrative and exemplary of embodiments and is made to provide an enabling disclosure of the exemplary embodiments. The disclosure is not intended to be construed to limit the embodiments of the invention or otherwise to exclude any other such embodiments, adaptations, variations, modifications and equivalent arrangements. The scope of the invention is defined by the appended claims.

## Claims

1. A method of analysing radiochemical purity and chemical impurities in a radiopharmaceutical composition, using reversed phase HPLC, wherein the radiopharmaceutical composition comprises a compound or a pharmaceutically acceptable salt thereof, labelled with [¹⁸F], and wherein said method comprises:
(a) providing a sample of the radiopharmaceutical composition to the HPLC column while the mobile phase is water, or water and organic solvent, or a neutral pH buffer, or a neutral pH buffer and organic solvent; and subsequently
(b) providing a different mobile phase comprising an acid modifier, water and organic solvent to the HPLC column.

2. A method according to claim 1, wherein the mobile phase in step (a) is water and organic solvent.

3. A method according to claim 1 or claim 2, wherein the organic solvent is selected from acetonitrile or methanol.

4. A method according to any one of claims 1 to 3, wherein the mobile phase in step (a) and/or step (b) comprises one or more organic solvents.

5. A method according to any one of claims 1 to 4, wherein the one or more organic solvents are the same in step (a) and step (b).

6. A method according to any one of claims 1 to 5, wherein the proportion of organic solvent in step (a) is between 10% and 60%.

7. A method according to any one of claims 1 to 6, wherein the proportion of organic solvent in step (b) is between 1% and 100%.

8. A method according to any one of claims 1 to 7, wherein the proportion of organic solvent is increased on a gradient in step (b); for example, wherein the increase in the proportion of organic solvent in step (b) is carried out in steps or as a continuous increase.

9. A method according to any one of claims 1 to 8, wherein the proportion of acid modifier in step (b) is at least 0.1%.

10. A method according to any one of claims 1 to 9, wherein the acid modifier in step (b) is selected from trifluoroacetic acid (TFA), formic acid, acetic acid, phosphoric acid, citric acid, and one or more acidic phosphate buffers, or a combination thereof.

11. A method according to any one of claims 1 to 10, wherein the acid modifier in step (b) is trifluoroacetic acid (TFA).

12. A method according to any one of claims 1 to 11, wherein the radiopharmaceutical composition (i) further comprises ethanol and cyclodextrin; and/or (ii) further comprises ascorbic acid.

13. A method according to any one of claims 1 to 12, wherein the concentration of buffer in mobile phase A is between 0.1 and 100 mM.

14. A method according to any one of claims 1 to 13, wherein the radiopharmaceutical composition is for use in Positron Emission Tomography (PET).

15. A method according to any one of claims 1 to 14, wherein the radiopharmaceutical is selected from [¹⁸F]FMAU (2'-deoxy-2'-[¹⁸F]fluoro-5-methyl-1-beta-D-arabinofuranosyluracil), [¹⁸F]FMISO([¹⁸F]Fluoromisonidazole), [¹⁸F]FHBG (9-(4-[¹⁸F]-Fluoro-3-[hydroxymethyl]butyl)guanine), [¹⁸F]AV-45, [¹⁸F]AV-19, [¹⁸F]AV-1, [¹⁸F]Flutemetamol, [¹⁸F]FES (Fluoroestradiol F18), [¹⁸F]flurpiridaz, [¹⁸F]K5, [¹⁸F]HX4, [¹⁸F]W372, [¹⁸F]VM4-037, [¹⁸F]CP18, [¹⁸F]ML-10, [¹⁸F]T808, [¹⁸F]T807, 2-[¹⁸F]fluoromethyl-L-phenylalanine, 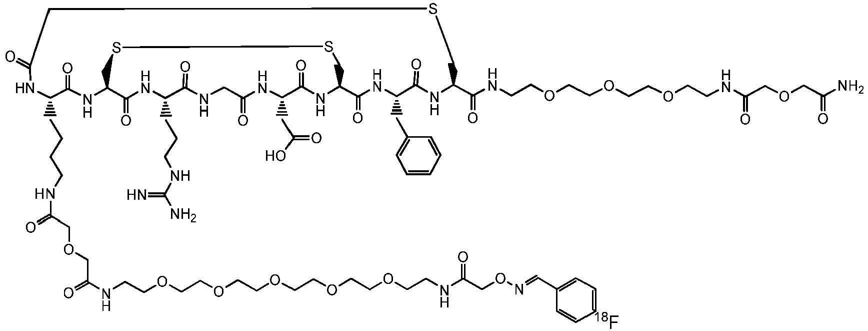 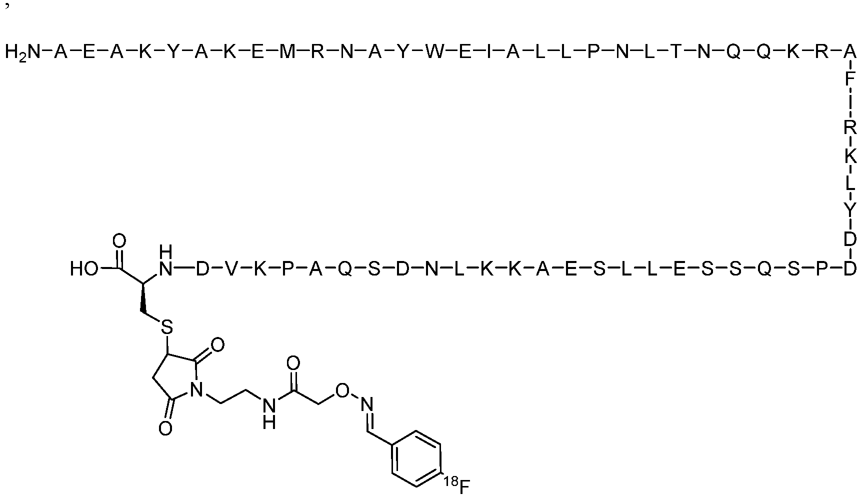 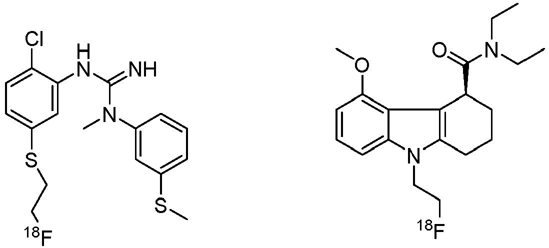 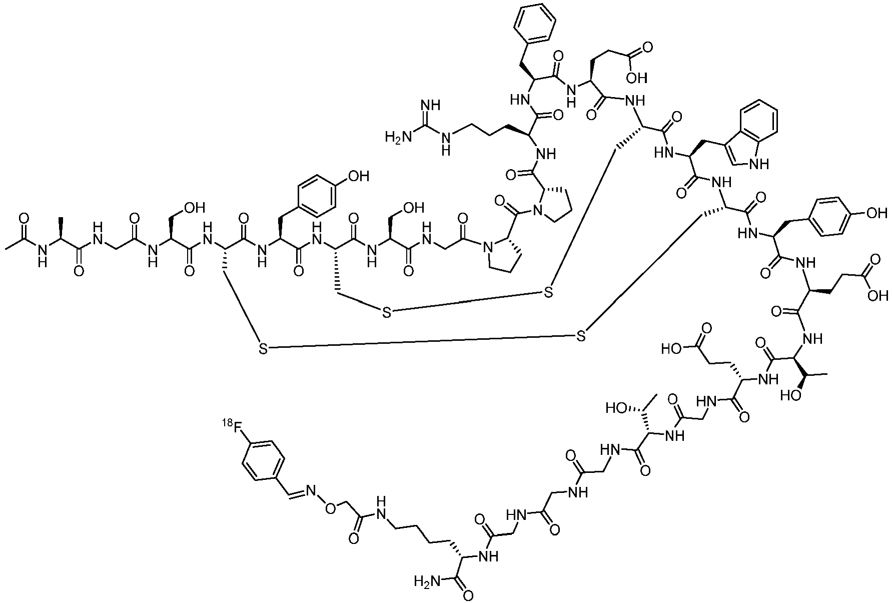 or a combination thereof.

16. A method according to any one of claims 1 to 15, wherein the radiopharmaceutical is [¹⁸F]flurpiridaz

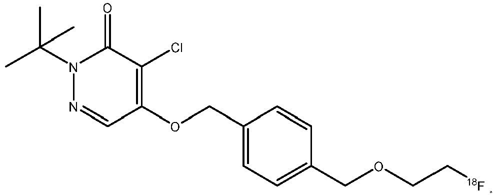

## Patentansprüche

1. Verfahren zum Analysieren radiochemischer Reinheit und chemischer Verunreinigungen in einer radiopharmazeutischen Zusammensetzung unter Verwendung von Umkehrphasen-HPLC, wobei die radiopharmazeutische Zusammensetzung eine mit [¹⁸F] markierte Verbindung oder ein pharmazeutisch unbedenkliches Salz davon umfasst und wobei das Verfahren Folgendes umfasst:
(a) die Bereitstellung einer Probe der radiopharmazeutischen Zusammensetzung an die HPLC-Säule, wobei es sich bei der mobilen Phase um Wasser oder um Wasser und organisches Lösungsmittel oder um einen Puffer mit neutralem pH-Wert oder um einen Puffer mit neutralem pH-Wert und organisches Lösungsmittel handelt, und anschließend
(b) die Bereitstellung einer anderen mobilen Phase, die einen Säuremodifikator, Wasser und organisches Lösungsmittel umfasst, an die HPLC-Säule.

2. Verfahren nach Anspruch 1, wobei es sich bei der mobilen Phase in Schritt (a) um Wasser und organisches Lösungsmittel handelt.

3. Verfahren nach Anspruch 1 oder Anspruch 2, wobei das organische Lösungsmittel aus Acetonitril und Methanol ausgewählt ist.

4. Verfahren nach einem der Ansprüche 1 bis 3, wobei die mobile Phase in Schritt (a) und/oder Schritt (b) ein oder mehrere organische Lösungsmittel umfasst.

5. Verfahren nach einem der Ansprüche 1 bis 4, wobei das eine oder die mehreren organischen Lösungsmittel in Schritt (a) und Schritt (b) gleich sind.

6. Verfahren nach einem der Ansprüche 1 bis 5, wobei der Anteil an organischem Lösungsmittel in Schritt (a) zwischen 10 % und 60 % beträgt.

7. Verfahren nach einem der Ansprüche 1 bis 6, wobei der Anteil an organischem Lösungsmittel in Schritt (b) zwischen 1 % und 100 % beträgt.

8. Verfahren nach einem der Ansprüche 1 bis 7, wobei der Anteil an organischem Lösungsmittel in Schritt (b) auf einem Gradienten erhöht wird, wobei zum Beispiel die Erhöhung des Anteils an organischem Lösungsmittel in Schritt (b) schrittweise oder kontinuierlich erfolgt.

9. Verfahren nach einem der Ansprüche 1 bis 8, wobei der Anteil an Säuremodifikator in Schritt (b) mindestens 0,1 % beträgt.

10. Verfahren nach einem der Ansprüche 1 bis 9, wobei der Säuremodifikator in Schritt (b) aus Trifluoressigsäure (TFA), Ameisensäure, Essigsäure, Phosphorsäure, Citronensäure und einem oder mehreren sauren Phosphatpuffern oder einer Kombination davon ausgewählt ist.

11. Verfahren nach einem der Ansprüche 1 bis 10, wobei der Säuremodifikator in Schritt (b) Trifluoressigsäure (TFA) ist.

12. Verfahren nach einem der Ansprüche 1 bis 11, wobei die radiopharmazeutische Zusammensetzung (i) weiterhin Ethanol und Cyclodextrin umfasst und/oder (ii) weiterhin Ascorbinsäure umfasst.

13. Verfahren nach einem der Ansprüche 1 bis 12, wobei die Konzentration des Puffers in der mobilen Phase A zwischen 0,1 und 100 mM liegt.

14. Verfahren nach einem der Ansprüche 1 bis 13, wobei die radiopharmazeutische Zusammensetzung zur Verwendung in der Positronenemissionstomographie (PET) bestimmt ist.

15. Verfahren nach einem der Ansprüche 1 bis 14, wobei das Radiopharmazeutikum aus [¹⁸F]FMAU (2'-Desoxy-2'-[¹⁸F]fluor-5-methyl-1-beta-D-arabinofuranosyluracil), [¹⁸F]FMISO ([¹⁸F]Fluormisonidazol), [¹⁸F]FHBG (9-(4-[¹⁸F]-Fluor-3-[hydroxymethyl]butyl)guanin), [¹⁸F]AV-45, [¹⁸F]AV-19, [¹⁸F]AV-1, [¹⁸F]Flutemetamol, [¹⁸F]FES (Fluorestradiol F18), [¹⁸F]Flurpiridaz, [¹⁸F]K5, [¹⁸F]HX4, [¹⁸F]W372, [¹⁸F]VM4-037, [¹⁹F]CP18, [¹⁸F]ML-10, [¹⁸F]T808, [¹⁸F] T807, 2-[¹⁸F] Fluormethyl-L-phenylalanin, 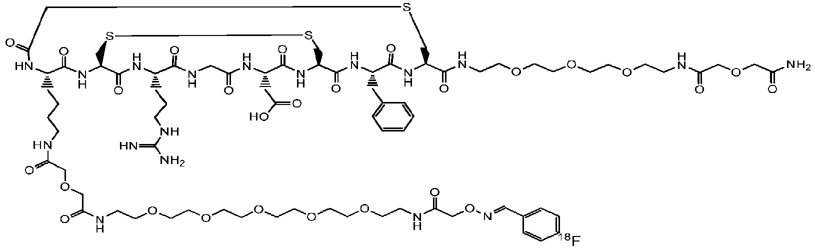 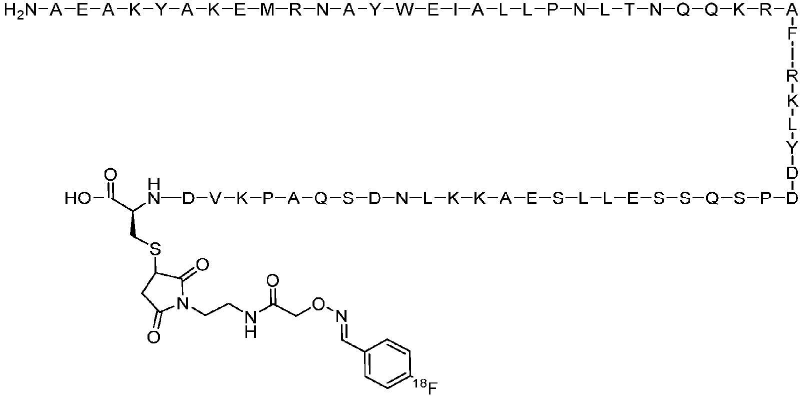 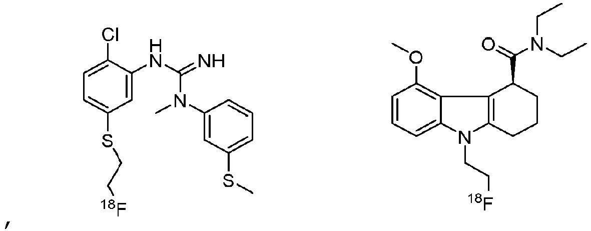 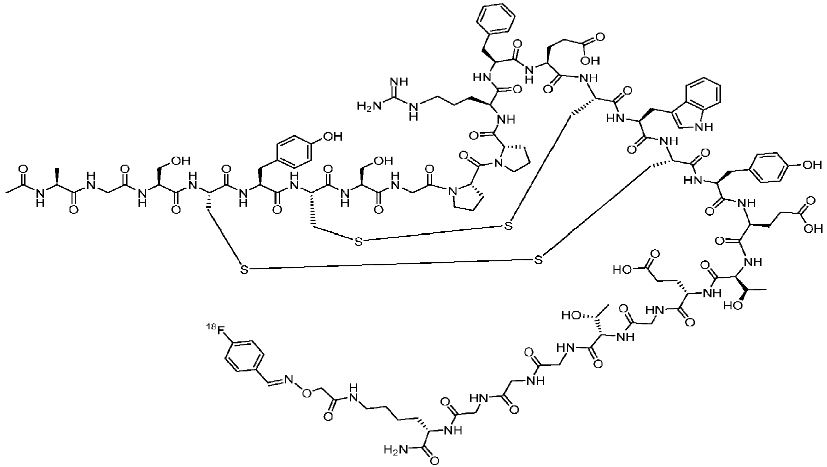 oder einer Kombination davon ausgewählt ist.

16. Verfahren nach einem der Ansprüche 1 bis 15, wobei es sich bei dem Radiopharmazeutikum um [¹⁸F]Flurpiridaz 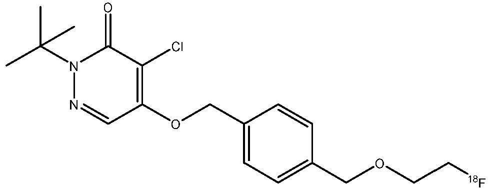 handelt.

## Revendications

1. Procédé d'analyse de la pureté radiochimique et des impuretés chimiques dans une composition radiopharmaceutique par CLHP en phase inverse, la composition radiopharmaceutique comprenant un composé ou un sel pharmaceutiquement acceptable de celui-ci, marqué par [¹⁸F], ledit procédé comprenant :
(a) l'introduction d'un échantillon de la composition radiopharmaceutique dans la colonne CLHP, la phase mobile étant de l'eau, ou de l'eau et un solvant organique, ou un tampon à pH neutre, ou un tampon à pH neutre et un solvant organique ; puis
(b) l'introduction d'une phase mobile différente comprenant un modificateur acide, de l'eau et un solvant organique dans la colonne CLHP.

2. Procédé selon la revendication 1, dans lequel la phase mobile de l'étape (a) est constituée d'eau et d'un solvant organique.

3. Procédé selon la revendication 1 ou 2, dans lequel le solvant organique est choisi parmi l'acétonitrile et le méthanol.

4. Procédé selon l'une quelconque des revendications 1 à 3, dans lequel la phase mobile de l'étape (a) et/ou de l'étape (b) comprend un ou plusieurs solvants organiques.

5. Procédé selon l'une quelconque des revendications 1 à 4, dans lequel le ou les solvants organiques sont identiques à l'étape (a) et à l'étape (b).

6. Procédé selon l'une quelconque des revendications 1 à 5, dans lequel la proportion de solvant organique à l'étape (a) est comprise entre 10 % et 60 %.

7. Procédé selon l'une quelconque des revendications 1 à 6, dans lequel la proportion de solvant organique à l'étape (b) est comprise entre 1 % et 100 %.

8. Procédé selon l'une quelconque des revendications 1 à 7, dans lequel la proportion de solvant organique est augmentée selon un gradient à l'étape (b) ; par exemple, lequel l'augmentation de la proportion de solvant organique à l'étape (b) est réalisée par paliers ou de manière continue.

9. Procédé selon l'une quelconque des revendications 1 à 8, dans lequel la proportion de modificateur acide à l'étape (b) est d'au moins 0,1 %.

10. Procédé selon l'une quelconque des revendications 1 à 9, dans lequel le modificateur acide à l'étape (b) est choisi parmi l'acide trifluoroacétique (TFA), l'acide formique, l'acide acétique, l'acide phosphorique, l'acide citrique et un ou plusieurs tampons phosphate acides, ou une combinaison de ceux-ci.

11. Procédé selon l'une quelconque des revendications 1 à 10, dans lequel le modificateur acide à l'étape (b) est l'acide trifluoroacétique (TFA).

12. Procédé selon l'une quelconque des revendications 1 à 11, dans lequel la composition radiopharmaceutique (i) comprend en outre de l'éthanol et de la cyclodextrine ; et/ou (ii) comprend en outre de l'acide ascorbique.

13. Procédé selon l'une quelconque des revendications 1 à 12, dans lequel la concentration de tampon dans la phase mobile A est comprise entre 0,1 et 100 mM.

14. Procédé selon l'une quelconque des revendications 1 à 13, dans lequel la composition radiopharmaceutique est destinée à être utilisée en tomographie par émission de positons (TEP).

15. Procédé selon l'une quelconque des revendications 1 à 14, dans lequel le produit radiopharmaceutique est choisi parmi [¹⁸F]FMAU (2'-désoxy-2'- [¹⁸F] fluoro-5-méthyl-1-bêta-D-arabinofuranosyluracile), [¹⁸F]FMISO ([¹⁸F]fluoromisonidazole), [¹⁸F] FHBG (9-(4-[¹⁸F]-fluoro-3-[hydroxyméthyl]butyl)guanine), [¹⁸F]AV-45, [¹⁸F]AV-19, [¹⁸F]AV-1, [¹⁸F] flutémétamol, [¹⁸F] FES (fluoroestradiol F18), [¹⁸F]flurpiridaz, [¹⁸F]K5, [¹⁸F] HX4, [¹⁸F]W372, [¹⁸F]VM4-037, [¹⁸F]CP18, [¹⁸F]ML-10, [¹⁸F] T808, [¹⁸F]T807, 2-[¹⁸F] fluorométhyl-L-phénylalanine, 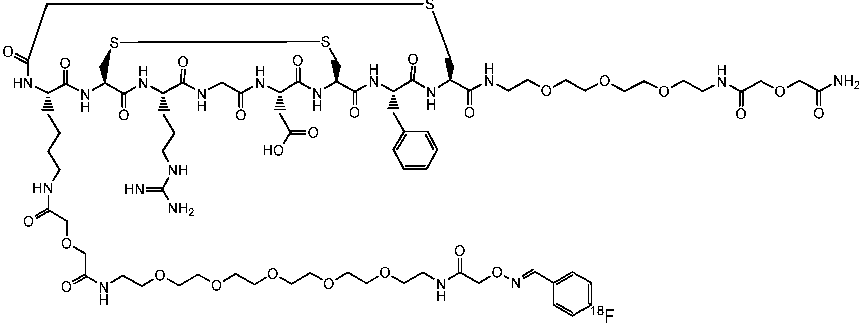 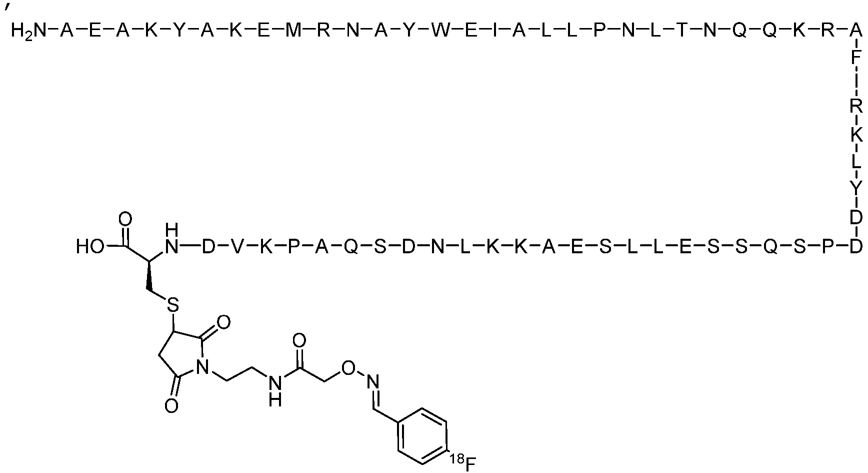 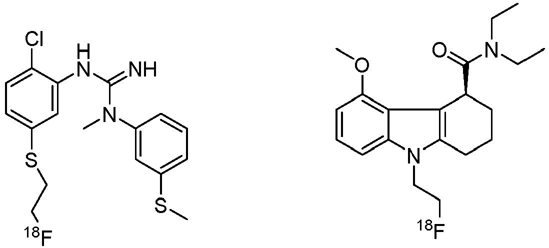 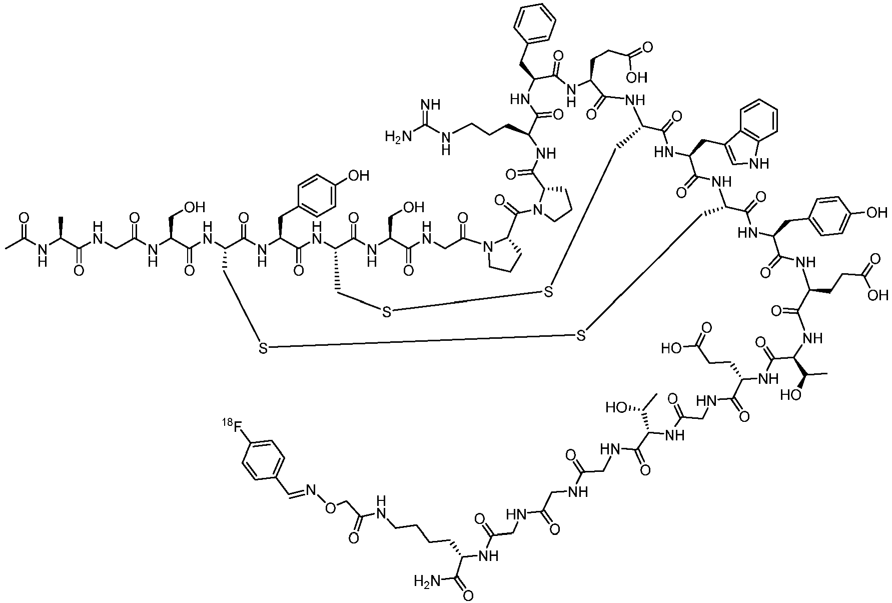 ou une combinaison de ceux-ci.

16. Procédé selon l'une quelconque des revendications 1 à 15, dans lequel le produit radiopharmaceutique est le
